# FASCICULE DE BREVET EUROPEEN

(11) **EP 0 798 310 B1**
(45) Date de publication et mention de la délivrance du brevet: **24.04.2002**
(21) Numéro de dépôt: 96402823.7
(22) Date de dépôt: 19.12.1996
(51) Int. Cl.: C07H 15/04

(54) **Procédé perfectionné d'oxydation des sucres**
Verbessertes Verfahren zur Oxidation von Zückern
Improved process for the oxidation of sugars

(30) Priorité: 21.12.1995 FR 9515269
(43) Date de publication de la demande: 01.10.1997
(73) Titulaire: Roquette Frères, 62136 Lestrem (FR)
(72) Inventeur: Fleche, Guy, 59190 Hazebrouck (FR)
(74) Mandataire: Boulinguiez, Didier

(56) Documents cités:
- EP-A- 0 532 370
- WO-A-95/07303
- US-A- 5 334 756

## Description

L'invention concerne un procédé d'oxydation des sucres.

De façon plus précise cette invention concerne un procédé d'oxydation des sucres par un hypohalite à l'aide d'un catalyseur constitué par un nitroxyl alcyle binaire ou tertiaire.

On sait que les sucres, et plus généralement leurs dérivés, peuvent être oxydés en milieu alcalin à l'aide d'hypohalites, en présence d'un catalyseur constitué par un nitroxyl alcyle binaire ou tertiaire.

Cette oxydation se traduit par l'apparition de fonctions acides carboxyliques en lieu et place des fonctions alcools primaires et également de la fonction hémiacétalique terminale des petits polymères de glucose par exemple.

Un tel procédé a été décrit dans la demande internationale de brevet WO 95/07303. Ce procédé, qui a été exemplifié sur les méthylglucosides, sur un disaccharide tel que le tréhalose et sur des polysaccharides tels que l'amidon, l'inuline et le pullulan, peut être également appliqué à des β-glucans tels que la cellulose, le curdlan ou le scleroglucan, et est également applicable à des hydrolysats ou à des dérivés de ces sucres.

Par ce procédé, l'amidon ou l'inuline, ou leurs hydrolysats, peuvent être oxydés en acides poly-α-glucuroniques ou poly-β-fructuroniques. Pour ce faire, une solution aqueuse alcaline contenant environ 7 à 15 grammes de matière sèche de sucre par litre d'eau, additionnée d'un catalyseur d'oxydation constitué par environ 0,1 à 2,5 % de 1-oxyl-2,2,6,6-tétraméthylpipéridine (appelé ci-après TEMPO) et de 20 % à 75 % de bromure de sodium en tant que co-oxydant, ces pourcentages étant exprimés par rapport au sucre, est soumise à l'action de l'eau de javel agissant comme oxydant.

Cette demande internationale de brevet WO 95/07303 enseigne également que pour être efficace, une telle réaction d'oxydation doit se dérouler à une température inférieure à 30°C et qu'il est hautement préférable de procéder à cette oxydation à une température comprise entre 0 et 5°C.

Dans ces conditions, il est indiqué que l'on peut obtenir par exemple des amidons oxydés à des taux proches du maximum théorique, à savoir que la quasi totalité des unités anhydroglucosyl de ces polymères sont transformées en unités anhydroglucuronyl. Les extrémités réductrices terminales hémiacétaliques du glucose ou des oligomères de glucose sont aussi oxydées en fonctions acides carboxyliques constituant ainsi un acide glucarique ou des glucuronyl-glucarates, à moins que ces extrémités ne soient protégées, par exemple, par méthylation. Cette dernière oxydation portant sur les extrémités réductrices non protégées, n'est, d'après l'enseignement de la demande de brevet en question, réelle que pour les hydrolysats d'amidon ou d'inuline de faible poids moléculaire.

On a trouvé, et c'est avant tout ce qui est à la base de la présente invention, que des taux d'oxydation tout aussi élevés pouvaient être obtenus, quel que soit le sucre à oxyder, si l'on utilisait des solutions aqueuses de sucres ou de leurs polymères contenant bien davantage que 15 grammes de sucre par litre d'eau.

En premier lieu, la présente invention concerne donc un procédé d'oxydation alcaline, et en milieu aqueux, de sucres possédant une ou plusieurs fonctions alcools primaires à l'aide d'hypohalites et en présence d'un catalyseur constitué par un nitroxyl alcyle binaire ou tertiaire, caractérisé par le fait que la concentration en sucrés du milieu aqueux est supérieure à 50 g/l, de préférence supérieure à 100 g/l et plus préférentiellement encore supérieure à 200 g/l.

On obtient ainsi une solution d'acides polyglucuroniques dans le cas de l'oxydation de polymères du glucose.

Dans le cas de l'oxydation d'aldoses monomères, on obtient ainsi les acides aldariques correspondant au monomère soumis à l'oxydation.

La Société Demanderesse a découvert que de façon surprenante, l'oxydation complète des sucres obtenue par l'oxydation catalysée par un nitroxyl alcyle binaire ou tertiaire, en milieu alcalin, à l'aide d'hypohalites, pouvait être assurée alors même que la concentration en sucres dans le milieu réactionnel était largement supérieure à 15 g/l.

On peut procéder à l'oxydation des sucres avec des concentrations allant jusqu'à 500 g/l et même davantage. La Société Demanderesse a découvert qu'en fait, ce sont surtout des problèmes de solubilité ou de viscosité qui limitent les concentrations auxquelles on peut procéder aux oxydations selon le procédé de l'invention.

On peut par exemple procéder à l'oxydation de substances de faible poids moléculaire telles que le xylose, l'arabinose, le glucose, le fructose, le mannitol, le sorbitol, le lactose, le lactitol, le maltitol, les hydrolysats d'amidon, ... à des concentrations largement supérieures à 100 g/l.

En outre, la Société Demanderesse a aussi remarqué qu'une telle oxydation pouvait être également conduite à des températures largement supérieures à 30°C, allant même jusqu'à 60°C. Ces températures élevées permettent d'augmenter la limite de solubilité des sucres, et de baisser la viscosité de leurs solutions, permettant ainsi d'accroître encore leur concentration.

Il est ainsi possible de procéder par exemple à 40°C, à l'oxydation de solutions contenant plus de 200 g/l de mannitol alors même qu'à une température de 0°C, la limite de solubilité de celui-ci serait largement dépassée.

Dans la mesure où l'on désire oxyder des sucres de nature polymérique tels que l'amidon, l'inuline, la cellulose, les pectines, les gommes ou leurs hydrolysats ou les produits en dérivant par estérification, éthérification, réticulation ...la limite de concentration des solutions à oxyder sera celle des contraintes imposées par la viscosité de ces solutions.

Cependant, cette limite sera d'autant plus haute que la température du milieu réactionnel sera plus élevée.

En second lieu, le procédé selon l'invention est donc caractérisé par le fait que l'oxydation est menée à une température supérieure à 30°C, de préférence supérieure à 40°C.

Comme les taux d'oxydation, de même que la sélectivité de l'oxydation vis-à-vis des alcools primaires ou des fonctions hémiacétaliques demeurent sensiblement les mêmes dans le procédé de l'invention que dans le procédé de l'art antérieur représenté essentiellement par la demande de brevet WO 95/07303, il est évident que l'accroissement des concentrations des sucres à oxyder selon le procédé de la présente invention résulte en une économie considérable pour ce qui concerne le volume des réacteurs nécessaires à la mise en oeuvre du procédé de l'invention.

De plus, le fait de travailler à des températures élevées permet de se dispenser de l'emploi de groupes frigorifiques extrêmement onéreux. La chaleur dégagée par ces réactions d'oxydation fortement exothermiques, d'autant plus importante que les milieux sont concentrés, peut être évacuée simplement à l'aide d'eau à la température ambiante.

Enfin, la Société Demanderesse a remarqué qu'un autre avantage des températures élevées,. en tout cas égales ou supérieures à 30°C, était que les vitesses d'oxydation des sucres s'en trouvaient accrues et qu'il devenait ainsi possible de diminuer les taux de catalyseurs, notamment les taux de TEMPO.

Cette augmentation considérable de l'efficacité du catalyseur à température élevée, fait qu'au moins pour ce qui concerne l'oxydation des sucres de faible masse moléculaire, il devient possible de diminuer très fortement, voire même de supprimer le co-catalyseur onéreux qu'est le bromure de sodium employé dans le procédé de l'art antérieur.

La sélectivité de l'oxydation ne s'en trouve que très légèrement affectée et on ne note ainsi que des traces d'oxydation portant sur des alcools secondaires.

Par contre, cette augmentation considérable de l'efficacité du catalyseur permet, à la différence de ce qui était enseigné dans le brevet WO 95/07303, d'oxyder également les extrémités hémiacétaliques des polymères de glucose de degré de polymérisation supérieur à 15, et de supprimer quasiment le pouvoir réducteur des hydrolysats d'amidon soumis à l'oxydation.

A la connaissance de la Société Demanderesse, de tels produits constitués de glucuronyl-glucarates contenant moins de 0,5 % de sucres réducteurs, n'existent pas.

On aura compris que le terme sucre(s) employé dans la présente invention, regroupe ici les oses, les uloses, les alditols, ainsi que tous les composés qui en dérivent tels que les acides ou poly-acides aldoniques, cétoaldoniques, uroniques, leurs dérivés acétylés, aminés, alcylés, carboxyméthylés, cationisés, ... et les polymères homogènes ou hétérogènes de ces sucres, pour peu que ces sucres soient porteurs d'au moins une fonction alcool primaire.

Le procédé de l'invention se montre par exemple particulièrement efficace sur des hydrolysats d'amidon et des hydrolysats d'amidon hydrogénés ou oxydés par d'autres procédés, et les produits qu'il permet d'obtenir montrent des performances très intéressantes dans les compositions lessivielles en raison de leurs propriétés complexantes conférées par les nombreuses fonctions carboxyliques qu'ils portent.

D'autres matériaux oxydables, utilisables dans le procédé de cette invention, peuvent également être constitués par des alcools primaires à chaîne hydrocarbonée tels que le méthanol, l'éthanol, le propanol, ... dans la mesure où ceux-ci sont solubles dans l'eau aux températures préférées du procédé de l'invention.

Pour mettre en oeuvre le procédé de l'invention, on dissout donc le sucre dans l'eau pour obtenir une concentration d'au moins 50 g/l.

Il est essentiel dans le procédé de l'invention que l'oxydation se déroule en milieu alcalin.

Le pH préféré, pour mener à bien la réaction d'oxydation du procédé de l'invention, est d'environ 9 à 13. L'apparition de fonctions acides au fur et à mesure de l'avancement de la réaction d'oxydation oblige, bien entendu, à introduire un alcali de façon continue dans le milieu réactionnel afin de maintenir ce pH.

Le brevet US-A-5334756 concerné un procédé pour la préparation de carboxylates, notamment d'alkylpolyglucosides, consistant à soumettre un alkylpolyglucoside contenant des groupements hydroxy primaires à une oxydation ménagée, mais en milieu faiblement alcalin, puisque le pH est compris entre 8,0 et 9,0 et de préférence entre 8,5 et 9,0.

Selon le procédé de l'invention, l'oxydation est conduite en présence d'un catalyseur constitué par une nitroxyl alcyle binaire ou tertiaire capable de libérer un cation nitrosonium qui est réduit en hydroxylamine lorsqu'une fonction alcool primaire est oxydée en acide carboxylique. Ce cation nitrosonium est régénéré in situ par un oxydant qui est constitué commodément par un hypochlorite.

Dans un mode de réalisation préféré de l'invention, le catalyseur est constitué par la 1-oxyl-2,2,6,6-tétraméthylpipéridine ou TEMPO et l'hypochlorite est l'eau de javel.

Le catalyseur peut être ajouté dans l'eau, soit avant, soit après l'addition du sucre.

La concentration du catalyseur doit être d'au moins 0,01 % par rapport au poids de sucre. Elle peut être d'autant plus faible que l'on travaille à température élevée. Cependant, on préfère employer des concentrations de TEMPO comprises entre 0,05 % et 1,5 %. Au plus on accroît la concentration de catalyseur, au plus le temps de réaction décroît. L'inverse est évidemment vrai.

La température de réaction , selon le procédé de l'invention, est d'au moins 30°C.

Des produits d'oxydation d'excellente qualité, ne contenant que quelques pour-cent d'oxydations ayant porté sur des alcools secondaires, sont obtenus à des températures allant jusqu'à environ 60°C ou même légèrement supérieures.

Ces oxydations non sélectives s'accroissent au fur et à mesure que s'élève la température.

La durée requise pour l'oxydation dépend à la fois de la température, du pH, et du pourcentage du catalyseur. De façon surprenante, la concentration en sucre du milieu réactionnel joue peu. De façon générale, les taux d'oxydation désirés, qui peuvent être compris entre 10 % et 100 %, et de manière plus générale entre 60 et 99 % des fonctions alcools primaires à oxyder sont obtenus en environ 1 minute à environ 2 heures, quoique selon les circonstances, et selon l'équipement des réacteurs d'oxydation (agitation, dimensionnement des échangeurs thermiques), on puisse procéder aux oxydations en des temps plus longs.

Ainsi, la durée requise pour l'oxydation est de loin inférieure à celle enseignée par le brevet US-A-5334756.

Lorsqu'on emploie par exemple de faibles taux de catalyseurs ou les températures les plus faibles, une durée d'oxydation plus longue peut être envisagée.

Des polymères plus ou moins lourds, pour peu qu'ils soient porteurs de fonctions alcools primaires, peuvent être utilisés dans le procédé de l'invention.

La Société Demanderesse a cependant remarqué que pour procéder à l'oxydation de sucres de masse moléculaire relativement faible tels que par exemple les monomères, dimères, et polymères de sucres simples ayant un degré de polymérisation inférieur à 20 environ, il n'était pas nécessaire d'utiliser un co-oxydant. Ceci va à l'encontre de l'enseignement de l'art antérieur.

Un procédé d'oxydation alcaline de monomères, dimères, et polymères de sucres simples ayant un degré de polymérisation inférieur à 20 environ selon l'invention, consiste donc à n'utiliser que l'eau de javel, sans addition de co-oxydant onéreux tel que le bromure de sodium.

L'oxydation du maltitol qui est un dimère, composé de glucose et de sorbitol liés en α, 1-4, est par exemple possible par la seule action de l'eau de javel sans addition de bromure.

On obtient ainsi, avec un rendement total, l'acide glucuronyl (α, 1-4) glucarique qui est un produit nouveau et qui montre de très intéressantes propriétés séquestrantes.

L'oxydation du maltitol pourra se dérouler par exemple selon le procédé de l'invention à une température de 40°C, avec un taux de TEMPO de 1,3 % et à une concentration en maltitol du sirop soumis à l'oxydation de 25 % et ce, sans bromure.

De manière générale, pour obtenir l'oxydation de toutes les fonctions alcools primaires d'un sucre, on préfère employer un léger excès d'oxydant, soit environ 2,2 moles de NaOCl par mole d'alcool primaire à oxyder, soit par exemple 6,6 moles de NaOCl par mole de maltitol à oxyder car le maltitol compte trois fonctions alcools primaires.

Cependant, la Demanderesse a remarqué que des sirops de glucose ou des maltodextrines d'un DE supérieur à 2 pouvaient être oxydés de façon quasi totale sans addition de co-catalyseurs, pour peu que l'on procède à leur oxydation à une température supérieure à 30°C.

Le procédé de la présente invention possède au moins quatre avantages par rapport au procédé proposé dans la demande de brevet WO 95/07303, dans lequel on procède à l'oxydation des sucres en milieu très dilué et très froid.

Premièrement, les taux de catalyseurs peuvent être abaissés ainsi qu'il a déjà été dit.

Deuxièmement, il n'est pas nécessaire, au moins lorsque l'on procède à l'oxydation de produits de faible masse moléculaire, d'utiliser des co-catalyseurs.

Troisièmement, il n'est pas nécessaire de recourir à l'utilisation de groupes frigorifiques.

Quatrièmement, il n'est pas nécessaire d'évaporer d'énormes quantités d'eau pour recouvrer les produits obtenus par le procédé de la présente invention.

Dans le procédé de la présente invention, on préfère procéder à l'addition progressive d'alcali au milieu. réactionnel de manière à maintenir constant le pH de ce milieu.

On pourrait aussi introduire en une seule fois, au début de la réaction, la quantité totale d'alcali que l'on estime nécessaire à l'accomplissement de celle-ci.

Le sucre et l'oxydant, ou le mélange oxydant-co-oxydant peuvent être introduits de façon continue dans le réacteur, mais on préfère les dissoudre en totalité dans l'eau dès le début de la réaction.

Lorsque la réaction d'oxydation est complète ou lorsqu'elle a atteint le seuil qu'on lui avait fixé et qui est fonction de la quantité relative d'oxydant mise en oeuvre, on constate l'arrêt de la consommation d'alcali ou la stabilisation du pH si l'on a introduit tout l'alcali en une seule fois au début de la réaction.

Le catalyseur peut alors être retiré du milieu réactionnel en procédant par exemple à une extraction par solvant, mais on peut aussi, d'une manière plus pratique, l'adsorber sur une colonne de charbon activé granulaire.

Après filtration, le milieu réactionnel ainsi débarrassé de son catalyseur et des fines de charbon, peut être traité par des techniques chromatographiques d'exclusion d'ions ou par des membranes de manière à en éliminer les sels minéraux.

S'il en est besoin, ces milieux réactionnels ainsi purifiés peuvent être concentrés et séchés. On peut aussi les soumettre à cristallisation pour en isoler des produits purs lorsque des sucres purs ont été soumis à l'oxydation.

D'autres détails de l'invention apparaîtront à la lecture des exemples qui suivent. Dans ces exemples, les concentrations en sucres sont données en pourcentage en poids de sucres par rapport au poids de solutions sucrées mises en oeuvre, les pourcentages de catalyseurs sont donnés en poids par rapport au poids de sucres secs mis en oeuvre.

### Exemple 1 :

Dans un réacteur agité et thermostaté muni d'une sonde de mesure du pH, on prépare une solution aqueuse à 50 % en poids de sorbitol par l'addition de 100 grammes de sorbitol à 100 ml d'eau déminéralisée. On porte cette solution à 45°C puis on y ajoute 0,8 g de TEMPO et 1,15l d'eau de javel à 48°C chlorométriques sous forme d'une solution préalablement diluée à 10 % et ajustée à pH 10,4 par de l'acide chlorhydrique.

On laisse se dérouler la réaction à la température de 45°C en faisant circuler de l'eau à 20°C dans la double enveloppe du réacteur et en y ajoutant de façon continue une solution de soude à 10 % de manière à maintenir le pH à 10,4.

Au bout de 20 minutes, on constate que la consommation de soude est devenue quasi nulle.

On percole alors le contenu du réacteur sur une colonne de charbon activé granulaire puis on élue cette colonne à l'eau de manière à en enlever le catalyseur.

L'analyse du produit obtenu montre que le mélange contient par rapport à la matière sèche :
- 33 % d'acide glucarique sous forme de son sel de sodium,
- 67 % de chlorure de sodium.

On ne détecte pas de produits de sur-oxydation de l'acide glucarique.

### Exemple 2 :

Dans le même réacteur, on prépare une solution aqueuse à 25 % en poids de mannitol par addition de 100 grammes de mannitol à 300 ml d'eau déminéralisée.

On porte cette solution à 55°C puis on y ajoute 1g de TEMPO et 1,15 l d'eau de javel à 48° chlorométriques sous forme d'une solution préalablement diluée à 25 % et ajustée à pH 10,6 par de l'acide chlorhydrique.

On laisse se dérouler la réaction à la température de 55°C en faisant circuler de l'eau à 20°C dans la double enveloppe du réacteur et en y ajoutant de façon continue une solution de soude à 10 %, de manière à maintenir le pH à 10,6.

Au bout de 30 minutes, on constate que la consommation de soude est devenue quasi nulle. On percole alors le contenu du réacteur sur une colonne de charbon activé granulaire de manière à en enlever le catalyseur puis on élue cette colonne à l'eau. On recueille 7 litres de solution contenant :
- 1,9 % d'acide mannarique sous forme de son sel de sodium,
- 4,1 % de chlorure de sodium.

On détecte des traces de produits de sur-oxydation de l'acide mannarique.

### Exemple 3 :

Dans le même réacteur, on prépare une solution aqueuse à 50 % en poids de maltitol par addition de 100 grammes de maltitol à 100 ml d'eau déminéralisée.

On porte cette solution à 35°C puis on y ajoute 0,3 % de TEMPO et 0,9 l d'eau de javel à 48° chlorométriques sous forme d'une solution préalablement diluée à 10 % et ajustée à pH 10,4 par de l'acide chlorhydrique.

On laisse se dérouler la réaction à la température de 45°C en faisant circuler de l'eau à 20°C dans la double enveloppe du réacteur et en ajoutant de façon continue une solution de soude à 25 %, de manière à maintenir le pH à 10,4.

Au bout de 40 minutes, on constate que la consommation de soude est devenue quasi nulle.

On percole alors le contenu du réacteur sur une colonne de charbon activé granulaire de manière à en enlever le catalyseur puis on élue cette colonne à l'eau.

L'analyse du produit obtenu montre que le mélange contient :
- 37 % d'acide glucuronyl-glucarique sous forme de son sel de sodium,
- 63 % de chlorure de sodium.

On ne détecte pas de produits de sur-oxydation de l'acide glucuronyl-glucarique.

On a procédé à l'élimination du sel présent dans le produit réactionnel brut par une technique chromatographique d'exclusion d'ions sur une résine cationique forte.

On a ainsi obtenu un produit ne titrant plus que 4% de chlorure de sodium. On a mesuré le pouvoir complexant de l'acide glucuronyl-glucarique ainsi purifié par potentiométrie avec une électrode de calcium et l'on a mesuré une valeur de 16 mg de calcium complexé par gramme d'acide glucuronyl-glucarique sous forme de son sel de sodium laissant présager pour ce produit d'excellentes performances en tant que builder entrant dans la formulation des compositions lessivielles.

### Exemple 4 :

Dans le même réacteur encore, on prépare une solution aqueuse à 20 % de GLUCIDEX^{R} 19 correspondant à 100 g de matières sèches. Ce produit est une maltodextrine commercialisée par la Demanderesse qui est obtenue par hydrolyse à l'α-amylase de l'amidon.

On porte cette solution à 35°C puis on y ajoute 1g de TEMPO et 0,75 l d'eau de javel à 48° chlorométriques sous forme d'une solution préalablement diluée à 50 % et ajustée à pH 10,3 par de l'acide chlorhydrique.

On laisse se dérouler l'opération à la température de 35°C en y ajoutant de façon continue une solution de soude à 10 % de manière à maintenir le pH à 10,3.

Au bout de 120 minutes, on constate que la consommation de soude est devenue quasi nulle.

On percole alors le contenu du réacteur sur une colonne de charbon activé granulaire, puis on élue cette colonne à l'eau.

L'analyse du produit obtenu montre que le mélange contient :
- 45 % d'acides glucarique, glucuronyl-glucarique et polyglucuronyl-glucarique sous forme de leurs sels de sodium,
- 55 % de chlorure de sodium.

Il possède un degré de polymérisation moyen d'environ 5 et sa teneur en sucres réducteurs libres, mesurée par la méthode de BERTRAND est de 0,3 % indiquant que dans les conditions particulières du procédé de l'invention, même les fonctions hémiacétaliques des polymères de glucose d'un degré de polymérisation supérieur à 20 sont oxydées et ce, malgré l'absence de co-oxydant.

### Exemple 5 :

Dans un fermenteur à cuve de verre de marque BIOLAFFITE, d'une contenance utile de 20 litres, on introduit 2995 grammes d'eau et 1591 grammes de soude pour former 4586 grammes d'une lessive de soude à 34,7 %.

On ajoute alors dans cette lessive de soude, 55 grammes d'anthraquinone-2-monosulfonate de sodium et 18,2 ml d'eau oxygénée à 110 volumes.

On aère alors le fermenteur avec un débit d'air de 20 litres par minute et en l'agitant à la vitesse de 1000 tours par minute.

Après avoir agité ce mélange à 25°C durant 30 minutes, on porte la température à 45°C. On ajoute alors lentement et de façon régulière en 3 heures 30 minutes, 18344 grammes d'un sirop de glucose obtenu par hydrolyse acide d'amidon de maïs, présentant une matière sèche de 50 % et un DE de 37. (Degré de polymérisation moyen égal à 2,7).

On fixe ensuite la température à 55°C et on poursuit l'agitation et l'aération durant 2 heures 30 minutes. La teneur en sucres réducteurs du milieu réactionnel s'est alors abaissée à 0,3 g/100 g de matières sèches de sirop de glucose. (Elle était au départ de 37 g/100 g).

Après avoir rectifié le pH de ce milieu réactionnel à 7, on le percole sur une colonne de charbon activé granulaire de manière à en ôter le sel de sodium de l'acide-2-anthraquinone monosulfonique.

Cette réaction de dégradation alcaline oxydative transforme le sirop de glucose composé de polyglucosyl-glucose en polyglucosyl-arabinonate.

Dans un réacteur thermostaté et agité, on introduit la totalité de ce sirop polyglucosyl-arabinonate sans le diluer.

Compte-tenu de la légère dilution consécutive à l'élimination du sel de sodium de l'acide-2-anthraquinone monosulfonique, la matière sèche sucrée de ce sirop s'élève à environ 38 %.

On porte ce sirop à la température de 45°C, puis on y ajoute 50 g de TEMPO et 58 l d'eau de javel à 48° chlorométriques sous forme d'une solution préalablement diluée à 25 % et ajustée à pH 10,4.

On laisse se dérouler la réaction à la température de 45°C en y ajoutant de façon continue une solution de soude à 10 % de manière à maintenir le pH à 10,4.

Au bout de 90 minutes, on constate que la consommation de soude est devenue quasi nulle.

On percole alors le contenu du réacteur sur une colonne de charbon activé granulaire de manière à en enlever le catalyseur, puis on élue cette colonne à l'eau.

On procède à l'enlèvement de la quasi totalité des sels minéraux présents dans cette solution par une technique d'exclusion d'ions employant des résines cationiques fortes.

Le produit obtenu alors a été concentré sous vide puis déshydraté par atomisation. Il a fourni une poudre blanche de glucuronyl-arabinarates.

On a utilisé cette poudre en substitution des polyacrylates dans une formule lessivielle à raison de 1 partie de glucuronyl-arabinarates ainsi enrichis, pour une partie de polyacrylates.

Non seulement les poudres obtenues ne colorent pas au stockage, mais elles présentent des qualités lessivielles très intéressantes puisque après avoir effectué 25 lavages consécutifs d'échantillons de tissus de coton et de coton/polyester, les indices de blancheur obtenus s'avèrent supérieurs au témoin polyacrylate.

En outre, le taux d'incrustations organiques s'avère significativement inférieur.

## Revendications

1. Procédé d'oxydation alcaline à un pH de 9 à 13, différent de 9, en milieu aqueux, de sucres possédant une ou plusieurs fonctions alcools primaires à l'aide d'hypohalites et en présence d'un catalyseur constitué par un nitroxyl alcyle binaire ou tertiaire, **caractérisé par le fait que** la concentration en sucres du milieu aqueux est supérieure à 50 g/l, de préférence supérieure à 100 g/l, et encore plus préférentiellement supérieure à 200 g/l.

2. Procédé d'oxydation alcaline de sucres selon la revendication 1, **caractérisé par le fait que** l'oxydation est menée à une température supérieure à 30°C, de préférence supérieure à 40°C.

3. Procédé d'oxydation alcaline de sucres selon les revendications 1 et 2, **caractérisé par le fait que** le catalyseur est la 1-oxyl-2,2,6,6-tétraméthylpipéridine.

4. Procédé d'oxydation alcaline de monomères, dimères, trimères et tétramères de sucres simples selon l'une quelconque des revendications 1 à 3, **caractérisé par le fait que** l'hypohalite est l'hypochlorite de sodium et qu'il est employé sans co-oxydant, notamment sans bromure de sodium.

5. Acide glucuronyl (α, 1-4) glucarique.

6. Glucuronyl-glucarates contenant moins de 0,5 % de sucres réducteurs.

7. Utilisation des produits obtenus par le procédé selon les revendications 1 à 4 dans les compositions lessivielles.

## Claims

1. Process for the alkaline oxidation at a pH of 9 to 13, different from 9 in aqueous medium, of sugars possessing one or a number of primary alcohol functional groups using hypohalites and in the presence of a catalyst composed of a binary or tertiary alkyl nitroxyl, **characterized in that** the concentration of sugars in the aqueous medium is greater than 50 g/l, preferably greater than 100 g/l and more preferentially still greater than 200 g/l.

2. Process for the alkaline oxidation of sugars according to Claim 1, **characterized in that** the oxidation is carried out at a temperature greater than 30°C, preferably greater than 40°C.

3. Process for the alkaline oxidation of sugars according to Claims 1 and 2, **characterized in that** the catalyst is 2,2,6,6-tetramethylpiperidin-1-oxyl.

4. Process for the alkaline oxidation of monomers, dimers, trimers and tetramers of simple sugars according to any one of Claims 1 to 3, **characterized in that** the hypohalite is sodium hypochlorite and that it is employed without a cooxidizing agent, in particular without sodium bromide.

5. Glucuronyl-α(1 4)-glucaric acid.

6. Glucuronyl glucarates containing less than 0.5% of reducing sugars.

7. Use of the products obtained by the process according to Claims 1 to 4 in detergent compositions.

## Patentansprüche

1. Verfahren zur alkalischen Oxidation von Zuckern mit einer oder mehreren primären Alkoholfunktionen in wässrigem Medium mit Hilfe von Hypohalogeniden und in Gegenwart eines aus binärem oder tertiärem Nitroxylalkyl bestehenden Katalysators bei einem pH von 9 bis 13 und nicht 9, **dadurch gekennzeichnet, dass** die Zuckerkonzentration des wässrigen Mediums höher als 50 g/l, vorzugsweise höher als 100 g/l und noch bevorzugter höher als 200 g/l ist.

2. Verfahren zur alkalischen Oxidation von Zuckern nach Anspruch 1, **dadurch gekennzeichnet, dass** die Oxidation bei einer Temperatur über 30°C, vorzugsweise über 40°C, durchgeführt wird.

3. Verfahren zur alkalischen Oxidation von Zuckern nach den Ansprüchen 1 und 2, **dadurch gekennzeichnet, dass** der Katalysator 1-Oxyl-2,2,6,6-tetramethylpiperidin ist.

4. Verfahren zur alkalischen Oxidation von Monomeren, Dimeren, Trimeren und Tetrameren von einfachen Zuckern nach einem der Ansprüche 1 bis 3, **dadurch gekennzeichnet, dass** das Hypohalogenid Natriumhypochlorit ist und dass es ohne Cooxidationsmittel, insbesondere ohne Natriumbromid, verwendet wird.

5. Glucuronyl(α, 1-4)glucarsäure.

6. Glucuronylglucarate, die weniger als 0,5 % reduzierende Zucker enthalten.

7. Verwendung der gemäß den Ansprüchen 1 bis 4 erhaltenen Produkte in Waschmittelzusammensetzungen.
